# EUROPÄISCHE PATENTSCHRIFT

(11) **EP 1 244 394 B1**
(45) Veröffentlichungstag und Bekanntmachung des Hinweises auf die Patenterteilung: **25.02.2009**
(21) Anmeldenummer: 00993582.6
(22) Anmeldetag: 18.12.2000
(51) Int. Cl.: A61B 19/00

(54) **REFLEKTORSYSTEM ZUR POSITIONSBESTIMMUNG**
REFLECTOR SYSTEM FOR DETERMINING POSITION
SYSTEME DE REFLECTEURS POUR LA DETERMINATION D'UNE POSITION

(30) Priorität: 23.12.1999 DE 19962376; 15.06.2000 DE 10029529
(43) Veröffentlichungstag der Anmeldung: 02.10.2002
(73) Patentinhaber: Northern Digital Inc., Waterloo, Ontario N2V 1C5 (CA)
(72) Erfinder: BRUNNER, Georg, 78465 Konstanz (DE); SCHMID, Manfred, 88634 Herdwangen (DE)
(74) Vertreter: Söltenfuss, Dirk Christian
(86) Internationale Anmeldenummer: PCT/EP2000/012892
(87) Internationale Veröffentlichungsnummer: WO 2001/047427

(56) Entgegenhaltungen:
- WO-A-97/23423
- WO-A-99/27839
- DE-A- 19 639 615
- US-A- 3 025 764
- US-A- 3 700 305
- US-A- 3 781 083

## Beschreibung

Die Erfindung betrifft ein Reflektorsystem für die Positionsbestimmung sowie ein entsprechendes Verfahren zur Positionsbestimmung.

Die aus dem Stand der Technik an sich bekannten optischen Navigationssysteme verwenden für die Positionsbestimmung entweder aktive optische Marken oder passive Marken. Als aktive Marken werden in der Regel Leuchtdioden (LED) eingesetzt. Die passiven Marken sind üblicherweise Kugeln mit einem reflektierenden Überzug oder retroreflektierende Folien.

Die DE 196 39 615 C2 offenbart ein sogenanntes Reflektoren-Referenzierungssystem für chirurgische und medizinische Instrumente, das aus einer Strahlungsquelle und einer Reflektorenbaugruppe mit zumindest zwei Reflektoren gebildet ist. Aus diesem Reflektorensystem wird ein Markersystem geschaffen, um eine Positionsbestimmung von Körperpartien oder von Instrumenten zu erzielen. Chirurgische Instrumente können beispielsweise mit einem Drei-Reflektoren-Adapter bestückt werden, der ein für dieses Instrument charakteristisches Reflexionsbild abgibt. Bei der Positionsbestimmung von zu behandelnden Körperpartien gibt jede Landmarke ein sowohl bei der diagnostischen Patientendatenerfassung als auch bei der nachfolgenden Behandlungsüberwachung nur für sich selbst charakteristisches Bild ab. Die Reflektoren bestehen aus Aluminiumkugeln und können mit einem reflektierenden Überzug versehen sein. Solche Kugeln geben aus allen Raumrichtungen betrachtet ein einheitliches Reflexionsbild ab, dabei müssen die Kugeln ausreichend groß sein, um durch die Kameras erfasst zu werden.

Die in der Medizintechnik auf Geräten oder Instrumenten angebrachten passiven Marken müssen häufig desinfiziert und sterilisiert werden. Die Sterilisation der medizinischen Instrumente kann z.B. mittels der Gassterilisation oder Dampfsterilisation erfolgen, die bis zu einem vollen Arbeitstag dauern kann, wodurch bei häufigem Einsatz mehrere Instrumentensätze eingekauft werden müssen. Nur dadurch ist gewährleistet, daß jederzeit sterilisierte Instrumente zur Verfügung stehen.

Um eine Reduzierung von Aufwand und Kosten zu erzielen, ist deshalb in der DE 196 39 615 C2 vorgeschlagen worden, die sehr teueren Marken auf den medizinischen Geräten und Instrumenten austauschbar anzubringen. Zu diesem Zweck sind die Marken als passive Reflektoren an einem Adapter angebracht, der seinerseits lösbar mit dem Instrument verbunden ist. Dadurch wird nun zwar die Austauschbarkeit der Marken ermöglicht. Das grundsätzlich Problem wird allerdings nicht beseitigt. Denn die bekannten passiven Marken überstehen die Reinigungsprozesse nur wenige Male und müssen bereits nach relativ wenigen Einsätzen durch neue Marken ersetzt werden.

Ferner offenbart das US-Patent Nr. 3,700,305 eine retroreflektierende Beschichtung, die aus einer Vielzahl von Mikroglaskugeln mit einem Durchmesser im Bereich von 10 bis 200 µm besteht, hinter denen ein dielektrisches Spiegelmaterial zur Reflexion einfallender Strahlen angeordnet ist.

WO-A-9927893 offenbart ein Reflektorsystem und ein Verfahren zur Positionsbestimmung mit den Merkmalen des Oberbegriffs von Anspruch 1 bzw. 13.

Ausgehend von dem vorgenannten Stand der Technik ist es Aufgabe der Erfindung, ein Reflektorsystem der eingangs genannten Art zu schaffen, bei dem passive Marken eingesetzt werden, die in einem großen Winkelbereich reflektieren und ein Vielfaches der bisher möglichen Reinigungsprozesse überstehen und nahezu beliebig oft autoklavierbar sind. Dabei müssen selbstverständlich auch die medizinischen Anforderungen erfüllt werden.

Erfindungsgemäß wird diese Aufgabe durch ein Reflektorsystem mit den Merkmalen des Anspruchs 1 bzw. durch ein Verfahren zur Positionsbestimmung mit den Merkmalen des Anspruchs 13 gelöst.

Vorteilhafte Weiterbildungen und Ausgestaltungen der Erfindung sind in den Unteransprüchen beschrieben.

Die Erfindung geht von der Erkenntnis aus, daß die bekannten reflektierenden Überzüge auf Kugeln oder anderen geometrischen Marken-Körpern nur eine begrenzte Lebensdauer besitzen. Dieser Nachteil wird mit der Erfindung dadurch vollständig beseitigt, daß erfindungsgemäß als passive Marke eine transparente Kugel eingesetzt wird, die einen solchen Brechungsindex aufweist, dass die vordere Fläche der Kugel, auf die die einfallenden Strahlen treffen, als Linse wirkt, deren Brennpunkt auf der hinteren Fläche der Kugel liegt, und so als Reflektor im UV-Bereich, im sichtbaren Bereich und im Infraroten Bereich (IR) wirkt.

Bei dem erfindungsgemäßen Reflektorsystem wird die Reflexion am Übergang von optisch unterschiedlich dichten Materialien ausgenutzt, was einem jeweils anderen Brechungsindex entspricht. Da es bei der so ausgebildeten transparenten Kugel keine Vorzugsrichtung gibt, retroreflektiert die Kugel in jedem Raumwinkel. Dadurch werden beispielsweise bei einem Brechungsindex von 1,9 ± 0,1 für Glas achsnahe Lichtstrahlen in die bleiche Richtung reflektiert. Die achsferne Lichtstrahlen werden dagegen unter einem bestimmten Raumwinkel reflektiert. Durch Änderung des Brechungsindexes läßt sich die in einem gegebenen Raumwinkel reflektierte Lichtleistung ändern und entsprechend maximieren.

Die Oberfläche der transparenten Kugel kann in Ausbildung der Erfindung ganz oder teilweise teildurchlässig verspiegelt sein. Die größte reflektierte Intensität der Lichtstrahlen wird bei einer Transmission von 67 % bzw. bei einer Reflexion von 33 % erreicht. Alternativ hierzu ist ein vorbestimmtes Winkelsegment des Reflektors komplett verspiegelt. Dadurch wird ein Lichtstrahl zwar nicht mehr in alle Raumrichtungen reflektiert, die reflektierte Intensität ist jedoch wesentlich größer als bei teildurchlässiger Verspiegelung.

Um eine gleichmäßigere Verteilung des reflektierten Lichtes zu bekommen, können die verspiegelten Oberflächen des Reflektors vorzugsweise diffus reflektierend ausgebildet sein.

Eine besondere Ausgestaltung der Erfindung wird dadurch erreicht, wenn zwischen der Kugel und der Reflexionsschicht (Verspiegelung) eine Schicht aus einem transparenten Werkstoff mit einem anderen Brechungsindex angeordnet ist. Der Öffnungswinkel des reflektierten Lichtes läßt sich dann durch den Brechungsindex der Kugel, durch den Brechungsindex der eingesetzten Zwischenschicht und durch die Dicke der Zwischenschicht beeinflussen.

Vorzugsweise wird das erfindungsgemäße Reflektorsystem in der Medizintechnik zur Bestimmung einer Position eines medizinischen Instrumentes oder Gerätes oder zur Bestimmung von Körperteilen von Patienten verwendet.

In der Zeichnung ist ein Beispiel der Erfindung dargestellt. Darin zeigen:
- Figur 1: ein medizinisches Instrument für die Neurochirurgie mit passiven Reflektoren in schematischer Darstellung;
- Figur 2: eine retroreflektierende Kugel mit einem Brechungsindex von etwa 1,9; und
- Figur 3: eine retroreflektierende Kugel mit einer teilverspiegelten Oberfläche.

Ganz allgemein versteht der Fachmann unter einer Reflexion in der Physik die Erscheinung, daß Teilchen oder Wellen, beispielsweise Schall oder Licht, an Grenzflächen, wie beispielsweise Luft und Glas, zurückgeworfen werden. Bei sehr glatter Grenzfläche gilt dann das Reflexionsgesetz, nach dem der Einfallswinkel des Lichtstrahles gleich dem Reflexionswinkel ist. Der einfallende, der reflektierte Strahl und das Einfallslot liegen in einer Ebene. Eine solche reguläre Reflexion erfährt ein Lichtstrahl an einem Spiegel bzw. einer verspiegelten Oberfläche eines Körpers. Sind die Oberflächenrauhigkeiten größer als die Wellenlänge der Strahlen, dann erfolgt eine sogenannte diffuse Reflexion, bei der die Strahlen in alle Raumrichtungen reflektiert werden.

Meist wird nur ein Teil der einfallenden Strahlung reflektiert, während der andere Teil absorbiert oder gebrochen wird. Beim Übergang von einem optisch dichteren zu einem dünneren Medium, beispielsweise von Glas in Luft oder umgekehrt Luft in Glas, wird nur ein Teil der einfallenden Strahlung reflektiert, während der andere Teil gebrochen wird. Grundsätzlich ist die Größe der Reflexion abhängig von dem Brechzahlunterschied der beiden Materialien, insbesondere hier von Glas. Das Licht läuft nicht geradlinig weiter, sondern wird an der Oberfläche entsprechend dem optischen Brechungsgesetz abhängig von den Brechzahlen gebrochen.

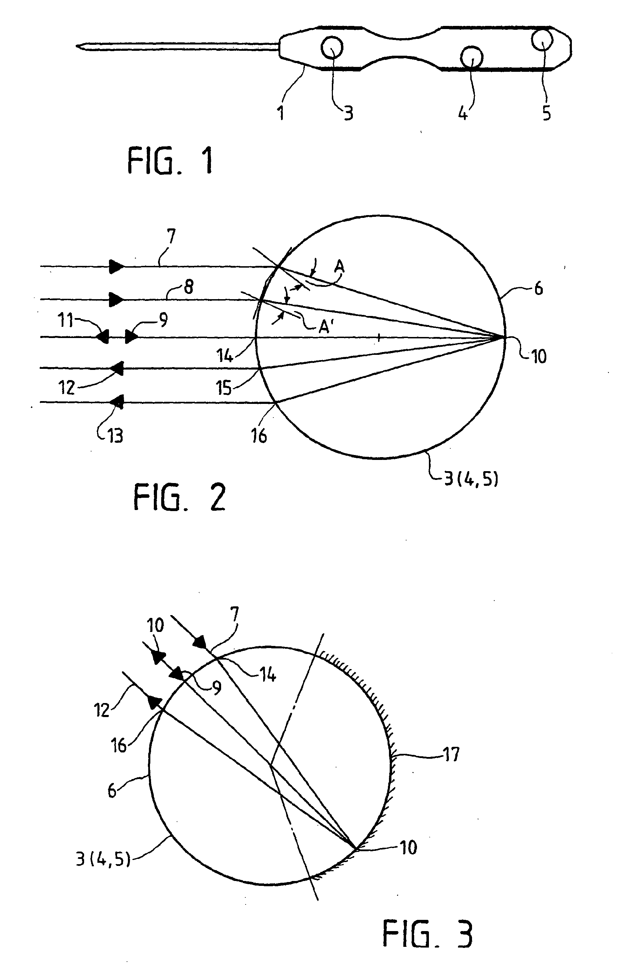

Das medizinische Instrument nach Figur 1 stellt ein Zeigeinstrument für neurochirurgische Eingriffe dar. Das medizinische Instrument 1 arbeitet vorzugsweise kabellos und besitzt insgesamt drei passive Reflektoren 3, 4 und 5. Selbstverständlich sind auch mehr oder weniger als drei Reflektoren und andere Anordnungen möglich.

Die Reflektoren 3, 4 und 5 sind gemäß den Figuren 2 und 3 retroreflektierend wirkende, transparente Kugeln. Als Werkstoff für die Kugel dient in bevorzugter Weise Glas. Eine elektromagnetische Strahlung, in der Regel ein Lichtstrahl, trifft auf die Kugeloberfläche 6 auf und wird an der Grenzschicht gebrochen, die zwischen der äußeren Luft und der Kugel aus dem Werkstoff Glas besteht. Die Luft hat einen Brechungsindex von 1, während hochbrechendes Glas einen Brechungsindex von nahezu 2 besitzt. Der Lichtstrahl tritt gemäß den Pfeilen 7, 8 und 9 in die Kugel 3, 4 und 5 ein. Die Kugeloberfläche 6 wirkt in diesem Fall wie eine Sammellinse. Die Lichtstrahlen 7 und 8 treffen auf die Kugeloberfläche auf und werden unter einem Winkel A bzw. A' abgelenkt und auf den gemeinsamen Endpunkt 10 in der Kugel gelenkt. Der achsnahe Lichtstrahl 9 verläuft in gerader Linie nach Eintritt in die Kugel auf den Endpunkt 10 zu. An diesem Endpunkt wird ein Teil des Lichtstrahles reflektiert und entsprechend den Pfeilen 11, 12 und 13 wieder zurückreflektiert und tritt an der Kugeloberfläche unter einem Winkel aus. Bei einer Spiegeloberfläche der Kugel ist der Einfallswinkel gleich oder nahezu gleich dem Ausfallwinkel. Das reflektierte Licht verläßt die Kugel wieder, wobei die Kugeloberfläche nun wiederum als Sammellinse wirkt.

In dem Beispiel der Figur 2 besteht die Kugel (Reflektor 3, 4, 5) aus Glas und weist keinerlei Überzüge oder Verspiegelungen auf. Das bedeutet, daß ein Teil der in die Kugel einfallenden Lichtstrahlen am Endpunkt 10 aus der Kugel austritt. Ein anderer und im Regelfall kleinerer Teil der Lichtstrahlen 7, 8 und 9 wird reflektiert und tritt an den reflektierten Orten 14, 15 und 16 aus der Kugel wieder aus. Diese reflektierten und aus der Kugel (Reflektor 3, 4 und 5) austretenden Lichtstrahlen werden von einem Empfangersystem, beispielsweise ein Kamerasystem, aufgenommen und entsprechend verwertet. Da die Kugel nach Figur 2 an keiner Stelle verspiegelt ist, können Lichtstrahlen aus allen Raumwinkeln auf die Kugeloberfläche auftreffen und retroreflektieren. Der dem Sender zugeordnete Öffnungswinkel für die Lichtstrahlen ist relativ groß. Wesentlich bei dieser Reflektorbauart ist es, daß die nach Reflexion aus dem Reflektor 3, 4 und 5 austretenden Lichtstrahlen so intensiv sind, daß sie durch den Empfänger sicher empfangen werden.

In dem Ausführungsbeispiel nach Figur 3 ist ein bestimmtes Winkelsegment 17 der Kugeloberfläche des Reflektors 3, 4 und 5 verspiegelt. Diese Maßnahme bewirkt, daß die Lichtstrahlen nur unter einem bestimmten Winkel in die Kugel einfallen können und reflektieren. Statt der Verspiegelung im Winkelsegment 17 ist auch eine diffus streuende Oberfläche denkbar. Obgleich Licht bei dieser Ausführung nicht mehr in alle Raumrichtungen reflektiert wird, so ist der Vorteil dieser Bauart die höhere Lichtintensität der austretenden Lichtstrahlen. Bei der diffus streuenden Oberfläche der Kugel erhält man eine gleichmäßigere Verteilung des reflektierten Lichtes.

Der nicht verspiegelte Teil der Kugeloberfläche kann natürlich entspiegelt sein.

Das Prinzip der Retroreflexion läßt sich im übrigen auch mit einer Linse und einem Hohlspiegel nachbilden. Wenn die Brennweite des Hohlspiegels *ƒ* ist, dann gilt näherungsweise für den Abstand von Linse zu Hohlspiegel der Wert 2*ƒ* und für die Brennweite der Linse ebenfalls 2*ƒ*. Durch Änderung des Abstandes oder durch Änderung der Brennweite der Linse läßt sich der Öffnungswinkel beeinflussen. Als Linse ist in dem vorgenannten Beispiel auch eine Fresnel-Linse einsetzbar.

Die Reflektoren 3, 4 und 5 unterliegen grundsätzlich dem physikalischen Gesetz der Brechung. Dringt nämlich ein Lichtstrahl aus einem optisch dünneren Medium, hier Luft, in ein optisch dichteres Medium, hier Glas, dann kommt es zu einer Brechung des Lichtstrahls, wobei das aus der Physik bekannte Brechungsgesetz gilt.

Es wird ausdrücklich hervorgehoben, daß diese neuartigen Reflektoren allgemein und vielfältig in der Technik einsetzbar sind und die geschilderte Anwendung in der Medizintechnik lediglich ein bevorzugtes Anwendungsgebiet darstellt.

## Patentansprüche

1. Reflektorsystem zur Positionsbestimmung, mit
einem Sender zum Aussenden paralleler Lichtstrahlen (7, 8, 9);
einem passiven Reflektor (3, 4, 5), der in der Form einer transparenten Kugel derart ausgebildet ist, dass auf einer Seite der Kugel einfallende Lichtstrahlen (7, 8, 9) an der gegenüber liegenden Seite der Kugel reflektiert und wieder von der einen Seite der Kugel ausgesendet (11, 12, 13) werden; und
einem Empfänger, der so ausgebildet und angeordnet ist, dass er die von dem passiven Reflektor (3, 4, 5) reflektierten Lichtstrahlen empfängt und die Position des passiven Reflektors (3, 4, 5) bestimmt,
**dadurch gekennzeichnet,**
**dass** der passive Reflektor (3, 4, 5) einen solchen Brechungsindex besitzt, dass eine vordere Fläche der Kugel als eine Linse wirkt, deren Brennpunkt auf einer hinteren Fläche der Kugel liegt.

2. Reflektorsystem nach Anspruch 1, bei welchem die Oberfläche der transparenten Kugel (3, 4, 5) wenigstens teilweise teildurchlässig verspiegelt ist.

3. Reflektorsystem nach Anspruch 1 oder 2, bei welchem ein Winkelsegment (17) der Oberfläche der transparenten Kugel (3, 4, 5) vorbestimmter Größe verspiegelt ist.

4. Reflektorsystem nach Anspruch 2 oder 3, bei welchem die verspiegelte Oberfläche der transparenten Kugel (3, 4, 5) diffus reflektierend ausgebildet ist.

5. Reflektorsystem nach einem der vorgenannten Ansprüche, bei welchem die transparente Kugel (3, 4, 5) aus einem Material gebildet ist, das nur für einen bestimmten Wellenlängenbereich transparent ist.

6. Reflektorsystem nach einem der vorgenannten Ansprüche, ferner mit einem Instrument (1), auf dem der passive Reflektor (3, 4, 5) angeordnet ist, wobei der Empfänger so angeordnet ist, dass er die von dem passiven Reflektor (3, 4, 5) reflektierten Lichtstrahlen empfängt und die Position des Instruments (1) bestimmt.

7. Reflektorsystem nach Anspruch 6, bei welchem das Instrument (1) ein medizinisches Instrument ist.

8. Reflektorsystem nach Anspruch 6 oder 7, bei welchem mehrere passive Reflektoren (3, 4, 5) beabstandet zueinander auf dem Instrument (1) angeordnet sind.

9. Reflektorsystem nach einem der vorgenannten Ansprüche, bei welchem der Empfänger eine Kamera ist.

10. Reflektorsystem nach einem der vorgenannten Ansprüche, bei weichem die transparente Kugel des passiven Reflektors (3, 4, 5) mit einer transparenten Schicht versehen ist, die einen anderen Brechungsindex als die transparente Kugel aufweist.

11. Reflektorsystem nach einem der vorgenannten Ansprüche, bei welchem der passive Reflektor (3, 4, 5) einen Brechungsindex von 1,9 ± 0,1 aufweist.

12. Verwendung eines Reflektorsystems nach einem der vorgenannten Ansprüche in der Medizintechnik zur Bestimmung einer Position eines medizinischen Instruments oder Geräts oder zur Bestimmung von Körperteilen von Patienten.

13. Verfahren zur Positionsbestimmung, mit
dem Anordnen mehrerer passiver Reflektoren (3, 4, 5) in einer vorbestimmten beabstandeten Beziehung an einem Gegenstand (1);
dem Richten paralleler Lichtstrahlen (7, 8, 9) von einem Sender auf wenigstens einige der mehreren passiven Reflektoren;
dem Erfassen von reflektierten Lichtstrahlen von den angestrahlten der passiven Reflektoren; und
dem Bestimmen der Position des Gegenstandes (1) basierend auf den erfassten reflektierten Lichtstrahlen von den angestrahlten der passiven Reflektoren, wobei jeder der passiven Reflektoren (3, 4, 5) als transparente Kugel mit einer ersten Seite und einer zweiten Seite ausgebildet ist,
**dadurch gekennzeichnet,**
**dass** der passive Reflektor einen solchen Brechungsindex besitzt, dass die erste Seite als eine Linse wirkt und die zweite Seite einen Brennpunkt bildet, sodass auf einer Seite der Kugel einfallende Lichtstrahlen (7, 8, 9) an der gegenüber liegenden Seite der Kugel reflektiert und wieder von der einen Seite der Kugel ausgesendet (11, 12, 13) werden.

## Claims

1. Reflector system for establishing positions, having
a transmitter for transmitting parallel light beams (7, 8, 9);
a passive reflector (3, 4, 5) which is in the form of a transparent sphere in such a manner that light beams (7, 8, 9) which are incident on one side of the sphere are reflected from the opposite side of the sphere and are transmitted (11, 12, 13) again from one side of the sphere; and
a receiver which is constructed and arranged in such a manner that it receives the light beams which are reflected from the passive reflector (3, 4, 5) and establishes the position of the passive reflector (3, 4, 5),
**characterised in that**
the passive reflector (3, 4, 5) has such a refractive index that a front face of the sphere acts as a lens whose focal point is at a rear face of the sphere.

2. Reflector system according to claim 1, wherein the surface of the transparent sphere (3, 4, 5) is at least partially coated with a partially permeable reflective coating.

3. Reflector system according to claim 1 or claim 2, wherein an angular segment (17) of the surface of the transparent sphere (3, 4, 5) of predetermined size is coated with a reflective coating.

4. Reflector system according to claim 2 or claim 3, wherein the surface of the transparent sphere (3, 4, 5) having the reflective coating is constructed so as to be reflective in a diffusive manner.

5. Reflector system according to any one of the preceding claims, wherein the transparent sphere (3, 4, 5) is constructed from a material which is transparent only with respect to a specific wavelength range.

6. Reflector system according to any one of the preceding claims, further having an instrument (1) on which the passive reflector (3, 4, 5) is arranged, with the receiver being arranged in such a manner that it receives the light beams which are reflected from the passive reflector (3, 4, 5) and establishes the position of the instrument (1).

7. Reflector system according to claim 6, wherein the instrument (1) is a medical instrument.

8. Reflector system according to claim 6 or claim 7, wherein a plurality of passive reflectors (3, 4, 5) are arranged with spacing from each other on the instrument (1).

9. Reflector system according to any one of the preceding claims, wherein the receiver is a camera.

10. Reflector system according to any one of the preceding claims, wherein the transparent sphere of the passive reflector (3, 4, 5) is provided with a transparent layer which has a refractive index different from that of the transparent sphere.

11. Reflector system according to any one of the preceding claims, wherein the passive reflector (3, 4, 5) has a refractive index of 1.9 ± 0.1.

12. Use of a reflector system according to any one of the preceding claims in medical technology in order to establish a position of a medical instrument or device or to establish the position of body parts of patients.

13. Method for establishing positions, having
the arrangement of a plurality of passive reflectors (3, 4, 5) in a predetermined spaced-apart relationship with respect to an object (1);
the orientation of parallel light beams (7, 8, 9) from a transmitter to at least some of the plurality of passive reflectors;
the detection of reflected light beams from those passive reflectors which are illuminated by light beams; and
the establishment of the position of the object (1) based on the detected, reflected light beams from those passive reflectors which are illuminated by light beams,
with each of the passive reflectors (3, 4, 5) being in the form of transparent spheres having a first side and a second side,
**characterised in that**
the passive reflector has such a refractive index that the first side acts as a lens and the second side constitutes a focal point so that light beams (7, 8, 9) which are incident on one side of the sphere are reflected from the opposite side of the sphere and are transmitted (11, 12, 13) again from one side of the sphere.

## Revendications

1. Système réflecteur pour la détermination de position, comprenant un émetteur servant à émettre des rayons lumineux parallèles (7, 8, 9) ;
un réflecteur passif (3, 4, 5) qui est configuré sous la forme d'une sphère transparente de telle manière que des rayons lumineux (7, 8, 9) tombant sur un côté de la sphère soient réfléchis sur la face opposée de la sphère et de nouveau émis par le premier côté de la sphère (11, 12, 13) ; et
un récepteur qui est configuré et disposé de telle manière qu'il reçoive les rayons lumineux réfléchis par le réflecteur passif (3, 4, 5) et qu'il détermine la position du réflecteur passif (3, 4, 5) ;
**caractérisé en ce que**
le réflecteur passif (3, 4, 5) possède un indice de réfraction tel qu'une face avant de la sphère se comporte comme une lentille dont le foyer se trouve sur une face arrière de la sphère.

2. Système réflecteur selon la revendication 1, dans lequel la surface de la sphère transparente (3, 4, 5) est métallisée de façon à être au moins en partie partiellement transparente.

3. Système réflecteur selon la revendication 1 ou 2, dans lequel un secteur angulaire (17) de la surface de la sphère transparente (3, 4, 5) d'une grandeur déterminée est métallisé.

4. Système réflecteur selon la revendication 2 ou 3, dans lequel la surface métallisée de la sphère transparente (3, 4, 5) est réfléchissante et diffusante.

5. Système réflecteur selon une des revendications précédentes, dans lequel la sphère transparente (3, 4, 5) est faite d'une matière qui est transparente seulement pour une certaine plage de longueurs d'onde.

6. Système réflecteur selon une des revendications précédentes, comprenant en outre un instrument (1) sur lequel le réflecteur passif (3, 4, 5) est disposé, le récepteur étant disposé de telle manière qu'il reçoive les rayons lumineux réfléchis par le réflecteur passif (3, 4, 5) et qu'il détermine la position de l'instrument (1).

7. Système réflecteur selon la revendication 6, dans lequel l'instrument est un instrument médical.

8. Système réflecteur selon la revendication 6 ou 7, dans lequel plusieurs réflecteurs passifs (3, 4, 5) sont disposés espacés les uns des autres sur l'instrument (1).

9. Système réflecteur selon une des revendications précédentes, dans lequel le récepteur est un appareil photographique.

10. Système réflecteur selon une des revendications précédentes, dans lequel la sphère transparente du réflecteur passif (3, 4, 5) est munie d'une couche transparente qui présente un indice de réfraction différent de celui de la sphère transparente.

11. Système réflecteur selon une des revendications précédentes, dans lequel la sphère transparente du réflecteur passif (3, 4, 5) présente une indice de réfraction de 1,9 ± 0,1.

12. Utilisation d'un système réflecteur selon une des revendications précédentes dans la technique médicale pour la détermination d'une position d'un instrument ou appareil médical ou pour la détermination de parties du corps de patients.

13. Procédé pour la détermination de position, comprenant les phases consistant à :
disposer plusieurs réflecteurs passifs (3, 4, 5) dans des relations d'espacement déterminées sur un objet (1) ;
projeter des rayons lumineux parallèles (7, 8, 9) issus d'un émetteur sur un au moins de certains des réflecteurs passifs,
détecter les rayons lumineux réfléchis par les réflecteurs passifs éclairés; et
déterminer la position de l'objet (1) en se basant sur les rayons lumineux réfléchis par les réflecteurs passifs éclairés et détectés, chacun des réflecteurs passifs (3, 4, 5) étant constitué par une sphère transparente ayant un premier côté et un deuxième côté,
**caractérisé en ce que** le réflecteur passif possède un indice de réfraction tel que le premier côté se comporte comme une lentille et que le deuxième côté forme un foyer, de sorte que les rayons lumineux (7, 8, 9) tombant sur un côté sont réfléchis sur le côté opposé et sont émis de nouveau par le premier côté de la sphère (11, 12, 13).
